# EUROPÄISCHE PATENTSCHRIFT

(11) **EP 1 886 694 B1**
(45) Veröffentlichungstag und Bekanntmachung des Hinweises auf die Patenterteilung: **03.06.2009**
(21) Anmeldenummer: 06016694.9
(22) Anmeldetag: 10.08.2006
(51) Int. Cl.: A61K 45/06, A61K 31/569, A61K 31/567, A61K 9/16, A61K 9/20, A61P 15/18, A61P 15/12

(54) **Perorale Arzneiform mit Dienogest und Ethinylestradiol zur Kontrazeption**
Peroral formulation of dienogest and ethinylestradiol for contraception
Formulation pérorale contenant le diénogest et l'éthinylestradiole comme contraceptif

(43) Veröffentlichungstag der Anmeldung: 13.02.2008
(73) Patentinhaber: Bayer Schering Pharma Aktiengesellschaft, 13353 Berlin (DE)
(72) Erfinder: Gerecke, Hagen, 07743 Jena (DE); Ladwig, Ralf, 07747 Jena (DE); Wiesinger, Herbert, 10781 Berlin (DE); Fricke, Sabine, 07749 Jena (DE); Buske, Alexander, 07745 Jena (DE)
(74) Vertreter: Cramer, Eva-Maria

(56) Entgegenhaltungen:
- WO-A-02/49675
- WO-A-03/091272
- WIEGRATZ I ET AL: "Effect of dienogest-containing oral contraceptives on lipid metabolism" CONTRACEPTION, GERON-X, INC., LOS ALTOS, CA, US, Bd. 65, Nr. 3, März 2002 (2002-03), Seiten 223-229, XP001207146 ISSN: 0010-7824
- JOHNSON M C: "Particle size distribution of the active ingredient for solid dosage forms of low dosage." PHARMACEUTICA ACTA HELVETIAE. 1972 AUG-SEP, Bd. 47, Nr. 8, August 1972 (1972-08), Seiten 546-559, XP008073300 ISSN: 0031-6865

## Beschreibung

### Technisches Gebiet

Die Erfindung betrifft eine perorale Arzneiform zur Kontrazeption, welche 17α-Cyanomethyl-17-β-hydroxyestra-4,9-dien-3on (Dienogest) gleich oder kleiner 2.0 mg und 17α-Ethinylestradiol (Ethinylestradiol) kleiner 0.030 mg enthält und wobei der kristalline Wirkstoff Dienogest mit einer durchschnittlichen Partikelgröße von 25 bis 270 µm enthalten ist.

### Stand der Technik

Orale empfängnisverhütende Mittel, bestehend aus einer Gestagenkomponente und einer Östrogenkomponente kamen erstmals in den frühen 60er Jahren auf den Markt. Drei wesentliche Eigenschaften charakterisieren das Profil der "Verhütungspille": kontrazeptive Sicherheit, eine sehr gute Zykluskontrolle sowie ein Minimum an Nebenwirkungen.

Seit Einführung hormoneller Kontrazeptiva ist die Forschung auf die Möglichkeit zur Schaffung von Arzneiformen gerichtet, die bei gleichbleibend guter kontrazeptiver Sicherheit und Zykluskontrolle unerwünschte Nebenwirkungen, wie beispielsweise arterielle und venöse Thrombosen und Beeinflussung des Kohlehydrat- und Fettstoffwechsels - hervorgerufen durch einen höheren Gehalt an Gestagen und Estrogen als zur Empfängnisverhütung notwendig - , reduziert. WO 98/004269 offenbart u. a. die orale Verabreichung einer Kombination von 250 µg - 4 mg Dienogest und 10 µg - 20 µg Ethinylestradiol zur Empfängnisverhütung. Um die wesentliche Verringerung des pro Zyklus verabreichten gesamten empfängnisverhütenden Steroids zu erreichen, während eine gute Zykluskontrolle beibehalten wird, verabreicht man die niedrig dosierte Gestagen/Östrogen-Kombination für 23 bis 25 Tage eines 28-tägigen Menstruationszyklus. Jedoch werden in der Patentschrift keine Ergebnisse und Angaben offenbart, die belegen, dass die erfinderische Idee auch zum Erfolg führt und welche Art der Freisetzung der Steroide angestrebt wird.

Es ist auch bekannt, dass bei niedrig dosierten oralen Kontrazeptiva unbedingt auf den täglich gleichen Zeitraum der Einnahme zu achten ist. Bei Nichteinhaltung dieses Zeitraumes wird die zur oralen Kontrazeption notwendig wirksame Wirkstoffkonzentration unterschritten und die orale Kontrazeption ist nicht mehr gewährleistet. Das bedeutet, dass die Anwenderin gehalten ist, ihren Einnahmezyklus sehr bewusst und mit großer Sorgfalt zu verfolgen.

Ferner ist aus der Fach- und Patentliteratur der Einfluss der Partikelgröße auf die pharmakokinetischen Eigenschaften eines Wirkstoffes bekannt.

Für Partikelgröße kann im folgenden Korngröße, für Homogenität der Wirkstoffverteilung auch Gehaltsgleichförmigkeit stehen.

Bauer, et al. Lehrbuch der pharmazeutischen Technologie, 6. Auflage, Wissenschaftliche Verlagsgesellschaft Stuttgart, 1999 beschreibt, dass bei einer Verabreichung einer Digoxin-Einzeldosis der mittleren Partikelgröße von 3,7 µm fast doppelt so hohe Plasmaspiegel gefunden wurden als nach Gabe eines Digoxin-Pulvers mit einer mittleren Partikelgröße von 22 µm.

W02003091272 beschreibt, das niedrig dosierte Formulierungen (Low dose Formulierungen) eine Homogenität der Wirkstoffverteilung (CUT) und eine gezielte Dissolutionskinetik erfordern. Dazu wird ein grobkörniges Wirkstoff-Kristallisat nach traditioneller Technologie in einer Strahlmühle mikronisiert.

Es werden durchschnittliche Partikelgrößen von 1.5 bis 5 µm erzielt.

Ferner führt W02003091272 aus, dass Partikelgrößen von 3 bis 25 µm, vorzugsweise 7 bis 15 µm für niedrig dosierte Formulierungen ausreichend sein können. Explizite Aussagen diesbezüglich werden nicht getroffen.

### Darstellung der Erfindung

Der Erfindung liegt die Aufgabe zugrunde, eine Möglichkeit zu schaffen, die eine Reduzierung der Steroidlast herkömmlicher Dienogest/Ethinylestradiol-Wirkstoffkombinationen für orale Kontrazeptiva realisiert und dabei eine kontrazeptive Wirksamkeit analog der konventionellen oralen Kontrazeptiva sichert.

Diese Aufgabe wird erfindungsgemäß durch eine feste perorale Arzneiform zur Kontrazeption gelöst, welche als Wirkstoffkombination Dienogest gleich oder kleiner 2.0 mg und Ethinylestradiol kleiner 0.030 mg enthält, wobei der kristalline Wirkstoff Dienogest mit einer durchschnittlichen Partikelgröße von 25 bis 70 µm enthalten ist.

Vorteilhafterweise beträgt die Partikelgröße des kristallinen Wirkstoffes Dienogest in der erfindungsgemäßen Arzneiform 40 bis 65 µm.

Auch kann die Wirkstoffkombination in der erfindungsgemäßen Arzneiform 0.75 bis 2.0 mg Dienogest und 0.015 bis 0.020 mg Ethinylestradiol betragen.

Der Wirkstoff Ethinylestradiol wird als ethanolische Lösung während der Granulation gesprüht oder in mikronisierter Form, vorzugsweise in der Tablettenmatrix/Tablettenkern eingesetzt.

Die Herstellung des Kristallisats des Wirkstoff Dienogest mit einer bestimmten Partikelgröße erfolgt aus einer übersättigten Lösung des Steroids, welche während der Kristallisation einem Naßmahlen unterzogen wird. Dabei wird die Primärkornsuspension erhalten.

Ferner sind andere Verfahren zur Herstellung von Dienogest mit der gewünschten Partikelgröße denkbar. Dazu zählen : Mahlung, Sichtung oder Siebung grobkörnigen Materials.

Die erfindungsgemäße perorale Arzneiform kann eine Tablette, eine Tablette mit einem Filmüberzug (Filmtablette) oder eine Tablette mit einer zuckerhaltigen Hülle (Dragee) sein. Auch sind unter die erfindungsgemäßen peroralen Arzneiformen zu zählen: Hartgelatinekapsel, Weichgelatinekapsel mit öligen oder wässrigen Suspensionen als Füllmasse oder andere perorale Suspensionen.

Die Freisetzung der Wirkstoffe, bzw. das Herauslösen dieser aus der Tablettenmatrix/Tablettenkern wird mit dem Dissolutionstest unter Verwendung von Wasser mit 37°C als Dissolutionmedium und 50 U/Min als Rührgeschwindigkeit bestimmt.

Die Bestimmung erfolgt nach Ph.Eur. mittels Blattrührerapparatur unter Verwendung von 1000 ml Wasser.

Eine bevorzugte erfindungsgemäße Arzneiform besteht darin, dass die Anzahl der Tagesdosiseinheiten, die die Kombination aus Dienogest und Ethinylestradiol enthalten, 21, 22, 23, 24, 25 oder 26 beträgt und die Anzahl der Tagesdosiseinheiten, die keinen Wirkstoff enthalten, 7, 6, 5, 4, 3 oder 2 im 28-tägigen Menstruationszyklus beträgt.

Es wurde gefunden, dass überraschenderweise die Verwendung des kristallinen Wirkstoffes Dienogest mit einer durchschnittlichen Partikelgröße von 25 bis 70 µm in der Matrix der peroralem Arzneiform die niedrige Dosierung der Wirkstoffkombination Dienogest/Ethinylestradiol und damit verbunden eine Reduzierung der Gesamtsteroidlast mit gleicher kontrazeptiver Wirksamkeit wie die konventionellen oralen Kontrazeptiva, Dienogest und Ethinylestradiol enthaltend, ermöglicht und eine Einnahmesicherheit gewährleistet wird. Die Einnahme kann z.B. morgens oder abends erfolgen ohne dabei unbedingt auf den exakten Zeitpunkt der Einnahme achten zu müssen. Jedoch sollte in etwa die gleiche Tageszeit eingehalten werden.

Die Bioverfügbarkeit der pharmazeutischen Wirkstoffe kann auch aus einer Kurve zur Plasmakonzentration als Fläche unter der Kurve (AUC) entnommen werden.

Ferner wurde gefunden, dass überraschenderweise mittels der erfindungsgemäßen peroralen Arzneiform eine Reduzierung der Gesamtsteroidlast (Reduzierung der Steroidlast an Dienogest und auch Reduzierung der Steroidlast an Ethinylestradiol) durch eine einphasige Wirkstofffreisetzung realisiert werden kann.

Die pharmazeutischen Anforderungen hinsichtlich der Homogenität der Wirkstoffverteilung wird durch die Parameter ihrer Partikelgrößenverteilung bestimmt.

Die Partikelgrößenverteilung des Dienogests wird über den Median der Partikelgrößenverteilung x₅₀ (Sucker, H. et al., Pharmazeutische Technologie, S. 16f, Georg Thieme Verlag Stuttgart, 1991) bestimmt.

Je höher die Partikelgröße eines Wirkstoffes ist, um so schwieriger ist ein gleichförmiger Gehalt des Wirkstoffes in den Tabletten zu erzielen. Die Gehaltsgleichförmigkeit kann entsprechend internationaler Pharmakopoen als relative Standardabweichung des Gehaltes einer Probe von Tabletten bestimmt werden. Werte unterhalb von 2 % entsprechen den pharmazeutischen Anforderungen hinsichtlich der Homogenität der Wirkstoffverteilung und gewährleisten eine gute technische Verarbeitbarkeit der pharmazeutischen Zusammensetzung. Das Arzneibuch lässt jedoch eine Standardabweichung bis zu 6,2 % zu, was in der Praxis jedoch zu Produktionsschwierigkeiten führen könnte.

Weitere Ausführungsformen, mit einer Anzahl an Tagesdosiseinheiten von 28 oder ein vielfaches von 28, beispielsweise 2 bis 3 mal 28 , die Dienogest gleich oder kleiner 2.0 mg und Ethinylestradiol kleiner 0.030 mg enthalten, sind möglich.

Auch ist die erfindungsgemäße perorale Arzneiform in der Hormonersatztherapie einsetzbar.

Andere Ausführungsformen mit Gestagenen, wie beispielsweise Levonorgestrel, Gestoden und weitere und/ oder Estrogenen, wie Estradiol, Estradiolvalerat , die allein oder in Kombination neben der Empfängnisverhütung auch zur Hormonsubstitution ( auch sequentiell) geeignet sind, sind möglich.

Es hat sich herausgestellt, dass die Wirkstoffkombination in der erfindungsgemäßen peroralen Arzneiform neben der Empfängnisverhütung antiandroge ne Eigenschaften besitzt und daher bei der Prophylaxe und Therapie von androgeninduzierten Störungen, insbesondere von Akne, verwendet werden kann.

### Darstellung der Zeichnungen

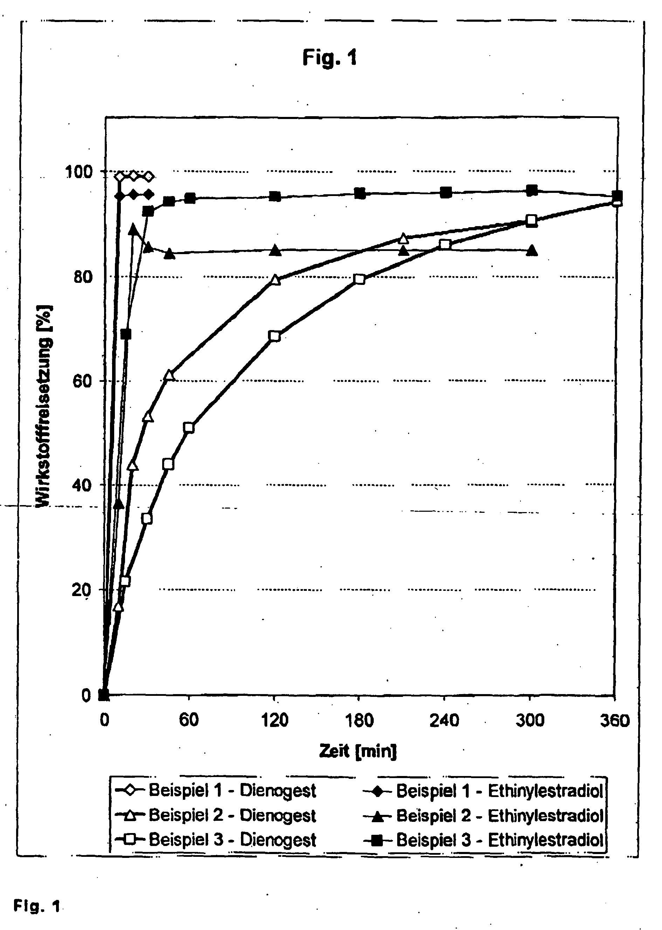

Die Erfindung wird unter Bezugnahme auf die beigefügten Zeichnungen genauer beschrieben, in der
in Fig.1 Kurven zur Wirkstofffreisetzung = In-vitro-Freisetzungsprofil von Dienogest und Ethinylestradiol nach den Beispielen 1 bis 3 aufgezeigt sind,
wobei Beispiel 1 Kurven zur Wirkstofffreisetzung von Dienogest und zur Wirkstofffreisetzung von Ethinylestradiol, hergestellt und gemessen nach Beispiel 1 für ein konventionelles Dragee, enthaltend 2 mg Dienogest und 0.030 mg Ethinylestradiol mit einer Dienogest-Partikelgröße von 3 µm sind - sie dienen als Vergleichskurven -
wobei Beispiel 2 Kurven zur Wirkstofffreisetzung von Dienogest und zur Wirkstofffreisetzung von Ethinylestradiol , hergestellt und gemessen nach Beispiel 2 für eine Tablette, enthaltend 1.5 mg Dienogest und 0.015 mg Ethinylestradiol mit einer Dienogest-Partikelgröße von 40 µm sind ;
wobei Beispiel 3 Kurven zur Wirkstofffreisetzung von Dienogest und zur Wirkstofffreisetzung von Ethinylestradiol , hergestellt und gemessen nach Beispiel 3 für eine Filmtablette, enthaltend 1.5 mg Dienogest und 0.015 mg Ethinylestradiol mit einer Dienogest-Partikelgröße von 65 µm sind.

Es hat sich herausgestellt, dass überraschenderweise mit der Vergrößerung der Partikelgröße von Dienogest, die schnelle Auflösung der Wirkverbindung aus der Zusammensetzung in vitro zu einer verzögerten Auflösung tendiert.

Als schnelle Auflösung wird die Auflösung von mindestens 70% des Wirkstoffes innerhalb von ca. 30 Minuten, vorzugsweise von mindestens 80% in etwa 20 Minuten definiert.

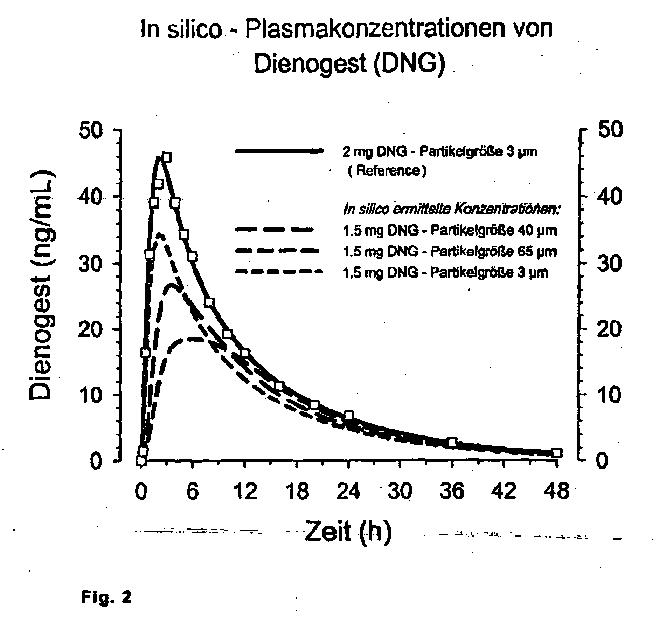

Basierend auf gemessenen humanen Plasmakonzentrationen nach der oralen Gabe von Dienogest (Dosis 2 mg, Partikelgröße 3 µm) wurden in silico Plasmakonzentrationen simuliert ( Fig. 2) sowohl unterschiedlicher Dosis mit gleicher Partikelgröße und gleicher Dosis mit unterschiedlicher Partikelgröße aufgezeigt.

Es ist ersichtlich, dass die maximale Plasmakonzentration Cmax von mikronisiertem Dienogest (Partikelgröße gleich 3 µm) nach 2 Stunden erreicht ist, unabhängig davon, in welcher Dosis Dienogest eingesetzt ist. Ferner ist ersichtlich, je größer die Partikelgröße bei gleicher Dosis an Dienogest ist, desto geringer ist Cmax, ohne dass dabei die Bioverfügbarkeit verringert wird.

Die Bioverfügbarkeit von Dienogest kann aus der Fläche unter der Kurve (AUC) für die Plasmakonzentration entnommen werden. Bei gleicher Dosis an Dienogest, aber zunehmender Partikelgröße halbiert sich nahezu Cmax von 100 % (3 µm) auf 78 % (40µm) und 54 % (65 µm). Gleichzeitig verlängert sich mit zunehmender Partikelgröße zeitlich tmax (Zeit bei Cmax) von 2 h (3 µm) auf 3.5 h (40µm) und 6 h (65 µm). Nach ca. 12 h ist der Absorptionsprozeß auch mit Dienogest in der maximalen Partikelgröße von 65 µm abgeschlossen. Danach wird Dienogest , unabhängig von der Korngröße, mit gleichen Geschwindigkeit aus dem Plasma eliminiert, und daher ähneln sich die Plasmakonzentrationen.

### Ausführungsbeispiele

### Beispiel 1

Valette ist ein konventionelles Dragee zur oralen Kontrazeption, enthaltend 0.030 mg Ethinylestradiol und 2.0 mg Dienogest in einem Tablettenkern, der mit einer zuckerhaltigen Hülle überzogen ist.

Als schwerlöslicher Wirkstoff wird Dienogest üblicherweise mikronisiert mit einer mittleren Teilchengröße von ca. 3 µm eingesetzt. Mit dieser Partikelgrößenverteilung wurde als optimale Dosis für eine sichere Ovulationshemmung 2 mg Dienogest ermittelt.

Das Ethinylestradiol wird als ethanolische Lösung während der Granulierung eingesprüht.

Es werden Tabletten mit folgender Zusammensetzung hergestellt:

| Kern: | |
|---|---|
| Dienogest | 2.000 mg |
| Ethinylestradiol | 0.030 mg |
| Laktose-Monohydrat | 28.720 mg |
| Maisstärke | 15.000 mg |
| Maltodextrin | 3.750 mg |
| Magnesiumstearat | 0.500 mg |

Alle Substanzen werden in geeigneter Weise granuliert und gemischt und anschließend zu Tablettenkernen Durchmesser 5 mm mit einer Masse von 50 mg gepresst.

| Hülle: | |
|---|---|
| Saccharose | 23.69340 mg |
| Glucosesirup | 1.65000 mg |
| Calciumcarbonat | 2.40000 mg |
| Povidon K25 | 0.15000 mg |
| Macrogol 35.000 | 1.35000 mg |
| Titandioxid | 0.74244 mg |
| Carnaubawachs | 0.01416 mg |

Die Substanzen werden in Wasser dispergiert und mit einem geeignetem Gerät auf die Tablettenkerne aufgesprüht bis zu einem Endgewicht der zuckerhaltigen Tablette von 80 mg.

Die Tabletten werden nach Ph. Eur., 4. Ausgabe, Grundwerk 2002, 2.9.3, auf ihre Wirkstofffeisetzung untersucht.

Folgende Bedingungen wurden eingehalten:
Medium: 1000 ml Wasser
Temperatur: 37 °C
Apparatur: Blattrührer 50 U/min
Probenahme: 7 ml mit Ersatz
Messung: HPLC

Es wurde folgende in-vitro Wirkstofffreisetzung gemessen :

| | |
|---|---|
| 10 min | 99.0 % Dienogest 95.2 % Ethinylestradiol |
| 20 min | 99.1 % Dienogest 95.5 % Ethinylestradiol |
| 30 min | 99.0 % Dienogest 95.5 % Ethinylestradiol |

Die Gleichförmigkeit des Gehaltes an Dienogest wurde an den Tabletten nach Ph.Eur. 2.9.40 ermittelt. Es ergab sich eine relative Standardabweichung des Gehaltes an Dienogest der Tabletten von 0.7 %.

### Beispiel 2

Herstellung und Dissolution und Blutspiegelkurven einer Dienogest/Ethinylestradiol-Filmtablette (1.5 mg Dienogest = DNG/0.015 mg Ethynylestradiol = EE) mit Dienogest im Kern der Filmtablette der mittleren Partikelgröße von 40 µm

Dienogest mit einem Median der Partikelgrößenverteilung von 40 µm wird verwendet. Der Wert wurde mittels Laserbeugung ermittelt. Ethinylestradiol wird als ethanolische Lösung während der Granulierung eingesprüht.

Es werden Filmtabletten mit folgender Zusammensetzung hergestellt:

| Kern: | |
|---|---|
| Dienogest | 1.500 mg |
| Ethinylestradiol | 0.015 mg |
| Laktose-Monohydrat | 53.835 mg |
| Maisstärke | 27.000 mg |
| Maltodextrin | 6.750 mg |
| Magnesiumstearat | 0.900 mg |

Alle Substanzen werden in geeigneter Weise granuliert und gemischt und anschließend zu Tablettenkernen Durchmesser 5.5 mm mit einer Masse von 90 mg gepresst.

| Hülle: | |
|---|---|
| Methocel | 3.000 mg |
| Macrogol | 0.600 mg |
| Talkum | 0.600 mg |
| Titandioxid | 1.700 mg |
| Eisenoxidpigment rot | 0.100 mg |

Die Substanzen werden in Wasser dispergiert und mit einem geeignetem Gerät auf die Tablettenkerne aufgesprüht bis zu einem Endgewicht der Filmtablette von 96 mg.

Die Tabletten werden nach Ph.Eur. 2.9.3 auf ihre Wirkstofffeisetzung untersucht.

Folgende Bedingungen wurden eingehalten:
Medium: 1000 ml Wasser
Temperatur: 37 °C
Apparatur: Blattrührer 50 U/min
Probenahme: 7 ml mit Ersatz
Messung: HPLC

Es wurde folgende in-vitro Wirkstofffreisetzung gemessen :

| | |
|---|---|
| 30 min | 53.3 % Dienogest 85.6 % Ethinylestradiol |
| 120 min | 79.5 % Dienogest 85.0 % Ethinylestradiol |
| 300 min | 90.5 % Dienogest 85.02 % Ethinylestradiol |

Die Gleichförmigkeit des Gehaltes an Dienogest wurde an den Tabletten nach Ph.Eur. 2.9.40 ermittelt. Es ergab sich eine relative Standardabweichung des Gehaltes an Dienogest der Tabletten von 3.3 %.

### Beispiel 3

Herstellung und Dissolution und Blutspiegelkurven einer Dienogest/Ethinylestradiol-Filmtablette (1.5 mg Dienogest = DNG/0.015 mg Ethynylestradiol = EE) mit Dienogest im Kern der Filmtablette der mittleren Partikelgröße von 65 µm

Dienogest mit einem Median der Partikelgrößenverteilung von 65 µm wird verwendet. Der Wert wurde mittels Laserbeugung ermittelt. Ethinylestradiol wird als ethanolische Lösung während der Granulierung eingesprüht.

Es werden Filmtabletten mit folgender Zusammensetzung hergestellt:

| Kern: | |
|---|---|
| Dienogest | 1.500 mg |
| Ethinylestradiol | 0.015 mg |
| Laktose-Monohydrat | 53.835 mg |
| Maisstärke | 27.000 mg |
| Maltodextrin | 6.750 mg |
| Magnesiumstearat | 0.900 mg |

Alle Substanzen werden in geeigneter Weise granuliert und gemischt und anschließend zu Tablettenkernen Durchmesser 5.5 mm mit einer Masse von 90 mg gepresst.

| Hülle: | |
|---|---|
| Methocel | 3.000 mg |
| Macrogol | 0.600 mg |
| Talkum | 0.600 mg |
| Titandioxid | 1.700 mg |
| Eisenoxidpigment rot | 0.100 mg |

Die Substanzen werden in Wasser dispergiert und mit einem geeignetem Gerät auf die Tablettenkerne aufgesprüht bis zu einem Endgewicht der Filmtablette von 96 mg.

Die Tabletten werden nach Ph.Eur. 2.9.3 auf ihre Wirkstofffeisetzung untersucht.

Folgende Bedingungen wurden eingehalten:
Medium: 1000 ml Wasser
Temperatur: 37 °C
Apparatur: Blattrührer 50 U/min
Probenahme: 7 ml mit Ersatz

Messung: HPLC

Es wurde folgende in-vitro Wirkstofffreisetzung gemessen :

| | |
|---|---|
| 30 min | 33.6 % Dienogest 92.3 % Ethinylestradiol |
| 120 min | 68.6 % Dienogest 95.1 % Ethinylestradiol |
| 300 min | 90.6 % Dienogest 96.3 % Ethinylestradiol |
| 360 min | 94.3 % Dienogest 95.2 % Ethinylestradiol |

Die Gleichförmigkeit des Gehaltes an Dienogest wurde an den Tabletten nach Ph.Eur. 2.9.40 ermittelt. Es ergab sich eine relative Standardabweichung des Gehaltes an Dienogest der Tabletten von 2.2 %.

## Patentansprüche

1. Perorale Arzneiform zur Kontrazeption, enthaltend 17α-Cyanomethyl-17-β-hydroxyestra-4,9-dien-3on (Dienogest) gleich oder kleiner 2.0 mg und 17a-Ethinylestradiol (Ethinylestradiol) kleiner 0.030 mg, **dadurch gekennzeichnet, dass** der kristalline Wirkstoff Dienogest mit einer durchschnittlichen Partikelgröße von 30 bis 270 µm enthalten ist.

2. Arzneiform nach Anspruch 1, **dadurch gekennzeichnet, dass** die Partikelgröße des kristallinen Wirkstoffes Dienogest 40 bis 65 µm beträgt.

3. Arzneiform nach einem der Ansprüche 1 bis 2, **dadurch gekennzeichnet, dass** die Wirkstoffkombination 0.75 bis 2.0 mg Dienogest und 0.015 bis 0.020 mg Ethinylestradiol enthält.

4. Arzneiform nach einem der Ansprüche 1 bis 3, **dadurch gekennzeichnet, dass** die Wirkstoffkombination 1.5 bis 2.0 mg Dienogest und 0.015 bis 0.020 mg Ethinylestradiol enthält.

5. Arzneiform nach einem der Ansprüche 1 bis 4, **dadurch gekennzeichnet, dass** die Anzahl der Tagesdosiseinheiten, die die Kombination von Dienogest und Ethinylestradiol enthalten, 21, 22, 23, 24, 25 oder 26 beträgt und die Anzahl der Tagesdosiseinheiten, die keinen Wirkstoff enthalten, 7, 6, 5, 4, 3 oder 2 beträgt.

## Claims

1. Peroral medicament form for contraception, comprising 17α-cyanomethyl-17β-hydroxyoestra-4,9-dien-3-one (dienogest) equal to or less than 2.0 mg and 17α-ethynyloestradiol (ethynyloestradiol) less than 0.030 mg, **characterized in that** the crystalline active compound dienogest is present with an average particle size of from 30 to 270 µm.

2. Medicament form according to Claim 1, **characterized in that** the particle size of the crystalline active compound dienogest is 40 to 65 µm.

3. Medicament form according to one of Claims 1 to 2, **characterized in that** the active compound combination comprises 0.75 to 2.0 mg of dienogest and 0.015 to 0.020 mg of ethynyloestradiol.

4. Medicament form according to one of Claims 1 to 3, **characterized in that** the active compound combination comprises 1.5 to 2.0 mg of dienogest and 0.015 to 0.020 mg of ethynyloestradiol.

5. Medicament form according to one of Claims 1 to 4, **characterized in that** the number of daily dose units which comprise the combination of dienogest and ethynyloestradiol is 21, 22, 23, 24, 25 or 26 and the number of daily dose units which comprise no active compound is 7, 6, 5, 4, 3 or 2.

## Revendications

1. Forme médicamenteuse perorale pour la contraception, contenant de la 17α-cyanométhyl-17β-hydroxyestra-4,9-dién-3-one (Dienogest) à raison de 2,0 mg ou moins et du 17α-éthynylestradiol (éthynylestradiol) à raison de moins de 0,030 mg, **caractérisée en ce que** la substance active cristalline Dienogest est contenue avec une grosseur de particules moyenne de 30 à 270 µm.

2. Forme médicamenteuse selon la revendication 1, **caractérisée en ce que** la grosseur des particules de la substance active cristalline Dienogest est de 40 à 65 µm.

3. Forme médicamenteuse selon l'une quelconque des revendications 1 à 2, **caractérisée en ce que** la combinaison de substances actives contient 0,75 à 2,0 mg de Dienogest et 0,015 à 0,020 mg d'éthynylestradiol.

4. Forme médicamenteuse selon l'une quelconque des revendications 1 à 3, **caractérisée en ce que** la combinaison de substances actives contient 1,5 à 2,0 mg de Dienogest et 0,015 à 0,020 mg d'éthynylestradiol.

5. Forme médicamenteuse selon l'une quelconque des revendications 1 à 4, **caractérisée en ce que** le nombre d'unités de dosage journalières qui contiennent la combinaison de Dienogest et d'éthynylestradiol, est de 21, 22, 23, 24, 25 ou 26 et le nombre d'unités de dosage journalières qui ne contiennent pas de substance active est de 7, 6, 5, 4, 3 ou 2.
